# EUROPEAN PATENT APPLICATION

(11) **EP 2 377 577 A1**
(43) Date of publication of application: **19.10.2011**
(21) Application number: 11161381.6
(22) Date of filing: 06.04.2011
(51) Int. Cl.: A61P 15/00, A61P 15/12, A61K 31/045, A61K 9/00, A61K 9/06

(54) **A benzyl alcohol mixture for treating premature ejaculation**

(30) Priority: 06.04.2010 US 321158 P
(71) Applicant: Panaxia Ltd, 50200 Beit-Dagan (IL)
(72) Inventor: Segal, Dadi, 61143 Tel Aviv (IL)
(74) Representative: Jones, Keith William

(57) **Abstract**

A penile desensitizing formulation for treating premature ejaculation, comprising benzyl alcohol at a concentration between 1% and 10% by weight of the total formulation, and additionally menthol at a concentration between 0.05% and 1% by weight of the total formulation, prepared as solution, gel, cream or ointment.

## Description

### CROSS REFERENCE TO RELATED APPLICATIONS

This application claims the benefit of U.S. Provisional Patent Application 61/321,158 filed on April 6th 2010, which is incorporated herein by reference.

### BACKGROUND

### 1. TECHNICAL FIELD

The present invention relates to the field of treating premature ejaculation, and more particularly, to treating premature ejaculation by desensitization.

### 2. DISCUSSION OF RELATED ART

Premature Ejaculation (PE) is a condition in which a man ejaculates earlier than he or his partner would like him to. Most men experience premature ejaculation at least once in their lives. PE affects 25%-40% of men in the United States.

Benzyl alcohol is an organic compound with the formula C₆H₅CH₂OH. It is a colorless liquid with a mild pleasant aromatic odor. Benzyl alcohol is produced naturally by many plants and is commonly found in fruits and teas. It is also found in a variety of essential oils including jasmine, hyacinth, and ylang-ylang

It is widely used in the industry for a variety of uses (solvent, buffer, carrier, preservative, etc.) due to its polarity, low toxicity, and low vapor pressure. It is partially soluble in water and completely miscible in alcohols and diethyl ether.

Benzyl alcohol is used as a general solvent for inks, paints, lacquers, and epoxy resin coatings. It is also a precursor to a variety of esters, used in the soap, perfume, and flavor industries. It is often added to intravenous medication solutions as a preservative due to its bacteriostatic and antipruritic properties. It is also used as a photographic developer.

### BRIEF SUMMARY

Embodiments of the present invention provide a penile desensitizing formulation comprising benzyl alcohol at a concentration between 1% and 10% by weight of the total formulation.

These, additional, and/or other aspects and/or advantages of the present invention are set forth in the detailed description which follows; possibly inferable from the detailed description; and/or learnable by practice of the present invention.

### DETAILED DESCRIPTION

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

Unexpectedly, Benzyl alcohol was found to be effective as the active ingredient to delay premature ejaculation, and even more so in a synergistic way, when combined with Menthol.

The present invention discloses a use of a mixture of Benzyl alcohol in any suitable liquid or semi solid vehicle as a penile desensitizing formulation for treating premature ejaculation.

A suitable concentration of Benzyl alcohol is in the range of 1-10%. Less than 1 % may not provide enough efficacy, whereas more than 10% might cause skin irritation and could lead to numbness and increase the incidence of side effects. 3%-7% is a more limited concentration range, whereas 5% was found to be the optimal in our tests. It was found that also various derivatives of benzyl alcohol (such as phenol, resorcinol, or other compound relating to benzyl alcohol) would provide similar effect to the one seen with benzyl alcohol and detailed below.

Benzyl alcohol may be mixed to be used as a cream, ointment, gel or solution but any liquid or semisolid vehicle will be appropriate. We have tested the formulation in cream, gel and liquid solution and all seem to provide the same efficacy.

In addition to benzyl alcohol, Menthol can be added to the mixture of Benzyl alcohol with a suitable liquid or semi solid vehicle. The relevant concentration of Menthol is 0.05%-1%. Concentration less than 0.05% would probably be too weak whereas more than 1% may cause skin irritation, a burning sensation or an intense odor. The concentration of benzyl alcohol is similar to that mentioned earlier.

In addition to benzyl alcohol, camphor may be added to the mixture of benzyl alcohol with a suitable liquid or semi solid vehicle. The relevant concentration of camphor is 0.25%-5%. Concentration less than 0.25% may be too weak whereas more than 5% may be too strong and could lead to burning sensation and an intense odor. The concentration of benzyl alcohol is similar to that mentioned earlier. The inventors have found 1% camphor to be an appropriate concentration.

In a trial which was conducted on 5 healthy, sexually active male subjects the following formulations were tested: Benzyl alcohol in concentrations: 1%, 3%, 5%, 7% and 10%. Each of the concentrations was tested in the following vehicles: aqueous alcoholic solution, gel, ointment and cream. For each formulation the participants were requested to rub the mixture on their genital organs until complete skin absorption and then wait for the full effect for 5 minutes. After the waiting period the participants were requested to perform manual stimulation of their genital organs and describe the desensitization effect of the mixture in a scale of 1-10. Mean average desensitization effect results of the different mixtures are given in **Table 1.**

**Table 1 - Effect of Benzyl alcohol in various concentrations and different vehicles on sexual desensitization of male subjects.**

| Vehicle | Benzyl alcohol concentration | Mean desensitization effect¹ |
|---|---|---|
| Aqueous alcoholic solution (1:1 water ethanol mixture) | 0% | 1.2 |
| | 1% | 3.2 |
| | 3% | 4.6 |
| | 5% | 6.2 |
| | 7% | 7.6 |
| | 10% | 8.0 |
| Aqueous gel (1% hydroxyethylcellulose gel) | 0% | 1.4 |
| | 1% | 2.8 |
| | 3% | 4.2 |
| | 5% | 5.6 |
| | 7% | 6.6 |
| | 10% | 7.4 |
| Cream | 0% | 1.0 |
| | 1% | 2.4 |
| | 3% | 3.6 |
| | 5% | 5.2 |
| | 7% | 6.2 |
| | 10% | 6.8 |
| Ointment | 0% | 1.4 |
| | 1% | 2.0 |
| | 3% | 3.2 |
| | 5% | 4.8 |
| | 7% | 5.8 |
| | 10% | 6.2 |
| ¹ Results are the average of individual scores given by each of the five participants in the trial. | | |

It can be seen from the study that each of the benzyl alcohol concentrations tested was effective as a desensitizer. There appears to be a direct correlation between the concentration of Benzyl alcohol in the tested mixture and the desensitization effect.

In another trial, menthol was added to the mixture and the same procedure that was described earlier was applied to assess the effect of menthol addition. Mean average desensitization effect results of the different mixtures are summarized in **Table 2**.

**Table 2 - Effect of mixtures of various Menthol concentrations and Benzyl alcohol gel on sexual desensitization of male subjects**

| Formulation | Menthol concentration | Mean desensitization effect¹ |
|---|---|---|
| 3% Benzyl alcohol in 1% Hydroxyethylcellulose gel | 0.0 | 4.2 |
| | 0.1 | 6.2 |
| | 0.3 | 7.2 |
| | 0.5 | 7.8 |
| Control - Menthol in 1% Hydroxyethylcellulose gel | 0.0 | 1.4 |
| | 0.1 | 1.2 |
| | 0.3 | 1.4 |
| | 0.5 | 1.6 |
| ¹Results are the average of individual scores given by each of the five participants in the trial. | | |

The synergistic effect of menthol addition to the benzyl alcohol gel can be observed. The sole use of menthol in 1% Hydroxyethylcellulose gel did not create a substantial desensitization effect, whereas combined with benzyl alcohol, menthol had a dramatic effect which was greater than the effect described in Table 1 for the same concentration of benzyl alcohol.

Further disclosed is a method of treating premature ejaculation, comprising rubbing any of the above mentioned formulations on genital organs until complete skin absorption, and the use of any of the above mentioned formulations for treating premature ejaculation.

In addition, camphor may be added to the benzyl alcohol and menthol mixture to enhance the desensitization effect.

In one embodiment, a small coin-sized amount of the formula is applied to the head of the penis for approximately 5-10 minutes, either during masturbation or just before sexual contact. Then the penis is massaged for approximately 5 minutes or until the gel is completely absorbed. The effect is felt within 1-5 minutes after applying the formula. One dosage program may comprise two applications per week in the first week, three applications per week in the second and third weeks, and four applications per week in the forth to sixth weeks. Afterwards, it is recommended to apply the formulation three times per week, and to apply the treatment every week.

In the above description, an embodiment is an example or implementation of the invention. The various appearances of "one embodiment", "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Furthermore, it is to be understood that the invention can be carried out or practiced in various ways and that the invention can be implemented in embodiments other than the ones outlined in the description above.

Meanings of technical and scientific terms used herein are to be commonly understood as by one of ordinary skill in the art to which the invention belongs, unless otherwise defined.

While the invention has been described with respect to a limited number of embodiments, these should not be construed as limitations on the scope of the invention, but rather as exemplifications of some of the preferred embodiments. Other possible variations, modifications, and applications are also within the scope of the invention. Accordingly, the scope of the invention should not be limited by what has thus far been described, but by the appended claims and their legal equivalents.

## Claims

1. A penile desensitizing formulation comprising benzyl alcohol at a concentration between 1% and 10% by weight of the total formulation, selected to inhibit premature ejaculation..

2. The formulation of claim 1, prepared as at least one of: an aqueous alcoholic solution, an aqueous gel, a cream, and an ointment.

3. The formulation of claim 1, prepared as an aqueous alcoholic solution and comprising a 1:1 water ethanol mixture.

4. The formulation of any of claims 1 to 3, wherein the benzyl alcohol is at a concentration between 3% and 7% by weight of the total formulation.

5. The formulation of claim 4, wherein the benzyl alcohol is at a concentration of 5% by weight of the total formulation.

6. The formulation of claim 1, 4 or 5, prepared as an aqueous gel and comprising a 1% hydroxyethyl cellulose gel.

7. The formulation of any of claims 1 to 6, further comprising menthol.

8. The formulation of claim 7, wherein the menthol is at a concentration between 0.05% and 1% by weight of the total formulation.

9. The formulation of claim 8, wherein the menthol is at a concentration between 0.1 % and 0.5% by weight of the total formulation.

10. The formulation of claim 7, further comprising a 1% hydroxyethyl cellulose gel.

11. The formulation of any of claims 7 to 10, wherein the benzyl alcohol is at a concentration of 3% by weight of the total formulation.

12. The formulation of any of claims 1 to 11, further comprising camphor.

13. The formulation of claim 12, wherein the camphor is at a 1% concentration by weight of the total formulation.

14. Use of benzyl alcohol according to the formulation of any of claims 1 to 13, for treating premature ejaculation.

15. The use according to claim 14, with two applications in a first week, three applications per week in a second and third weeks, and four applications per week in a forth to sixth weeks.
